Europäisches Patentamt

European Patent Office

Office européen des brevets

(19)

(11) Veröffentlichungsnummer: **0 335 189**
**A1**

(12) # EUROPÄISCHE PATENTANMELDUNG

(21) Anmeldenummer: **89104681.5**

(22) Anmeldetag: **16.03.89**

(51) Int. Cl.⁴: **B01F 5/02**

(30) Priorität: **26.03.88 DE 3810462**

(43) Veröffentlichungstag der Anmeldung:
**04.10.89 Patentblatt 89/40**

(84) Benannte Vertragsstaaten:
**AT BE CH DE ES FR GB GR IT LI LU NL SE**

(71) Anmelder: **DECHEMA Deutsche Gesellschaft für Chemisches Apparatewesen, Chemische Technik und Biotechnologie e.V.**
**Theodor-Heuss-Allee 25**
**D-6000 Frankfurt am Main 97(DE)**

(72) Erfinder: **Krämer, Peter, Dr.-Ing.**
**Ahornweg 12**
**D-6236 Eschborn 2(DE)**
Erfinder: **Bomberg, Andreas Rolf, Dipl.-Biol.**
**Marktplatz 5**
**D-6453 Seligenstadt(DE)**
Erfinder: **Gödelmann, Bernhard**
**Königsteiner Strasse 83**
**D-6230 Frankfurt am Main 80(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

(54) Verfahren zum Homogenisieren von Emulsionen und Vorrichtung zur Durchführung des Verfahrens.

(57) Verfahren zum Homogenisieren von Emulsionen durch Eindüsen von zwei Teilströmen der Emulsion in eine Homogenisierkammer mit einer Düsenaustrittsgeschwindigkeit von je mindestens 75 m/s, wobei die Düsen der Homogenisierkammer genau gegeneinander gerichtet, miteinander fluchtend und in geringem Abstand voneinander angeordnet sind, sowie Vorrichtung zur Durchführung dieses Verfahrens.

EP 0 335 189 A1

## Verfahren zum Homogenisieren von Emulsionen und Vorrichtung zur Durchführung des Verfahrens

:

Die vorliegende Erfindung betrifft ein Verfahren zum Homogenisieren von Emulsionen sowie die dafür geeignete Vorrichtung.

Als Emulsionen bezeichnet man die mehr oder weniger feine Verteilung einer Flüssigkeit in einer anderen, nicht moleculardispers mischbaren. Solche Emulsionen können, sofern sie nicht bereits natürlich vorkommen (z.B. Milch) auf mechanischem Wege durch Mischen und Zerkleinern der Tröpfchen der zu emulgierenden Phase und die damit verbundene Grenzflächenvergrösserung hergestellt werden. Als Hilfsmittel dafür sind u.a. schnell-laufende Rührwerke, Emulgierzentrifugen, Mischpumpen, Zerstäuber, Vibratoren und Ultraschallgeneratoren bekannt. Der Grad der Verteilung der einen Flüssigkeit (disperse Phase) in der anderen (äussere Phase) hängt entscheidend von den den Grenzflächen zuge führten Reibungs- und Scherkräften ab. Reicht für den gewünschten Zweck die Durchmischung der zu verteilenden Phasen nicht aus, kann man sogenannte Homogenisatoren einsetzen, um die noch zu grobe, polydisperse Emulsion zu zerkleinern.

Eine Methode bedient sich der Hochdruckhomogenisation, die ursprünglich aus der Milchverarbeitung stammt. Dabei wird die Emulsion mit einer Hochdruckkolbenpumpe in eine Ventileinheit gepumpt. Das Ventil öffnet erst nach Überschreiten eines über ein Hydrauliksystem je nach Anforderung einstellbaren Gegendrucks von etwa 150 - 350 bar. Die Emulsion wird gegen einen Prallring geschleudert und nach Umlenkung um 90° abgeleitet.

Ähnlich arbeiten Homogenisatoren mit Homogenisierköpfen, wie sie in DE-PS 310 267 beschrieben sind. Auch hier sind relativ hohe Betriebsdrukke notwendig.

Ebenfalls in Druckbereichen wie bei der Hochdruckhomogenisation arbeiten Vorrichtungen, wie sie aus US-PS 4 533 254 bekannt und im Handel sind. Die Spaltbreiten der rechteckigen Strömungskanäle liegen dort um 10μm x 1000μm und der Abstand der Austrittsöffnungen beträgt 0,2 mm.

Der Nachteil solcher Homogenisatoren liegt darin, dass einerseits hohe Betriebsdrucke (bis zu 1200 bar) erforderlich sind, und andererseits die Durchflussgeschwindigkeit häufig nur über einen apparativen Umbau variiert werden und deshalb zur Anpassung an geforderte Verfahrensergebnisse nur bedingt herangezogen werden kann. Hinzu kommt, dass die Ventileinheit bzw. die Homogenisierköpfe selbst sehr hohen Drucken und damit erheblichen Erosionen ausgesetzt sind und deshalb an die dafür zu verwendenden Werkstoffe hohe Anforderungen gestellt werden.

Ausserdem tritt eine Erwärmung der zu homogenisierenden Emulsion ein, die bei einem handelsüblichen Gerät nach Angaben des Herstellers im Bereich von 2,0 -2,4° C/100 bar liegt und in Einzelfällen bei hohen Betriebsdrücken zu einer Schädigung der Produkte führen kann.

Aus DE-OS 33 19 921 ist ein Verfahren zur Herstellung feinteiliger Dispersionen bekannt, bei dem die Homogenisierung durch eine nichtfördernde Rotor-Stator-Dispergiermaschine erfolgt. Die Anpassung an vorgegebene Verfahrensziele bedarf, soweit diese nur über die Änderung des Spalts zwischen Rotor und Stator zu erreichen sind, eines apparativen Umbaus.

Aus DE-OS 32 30 298 ist eine Vorrichtung bekannt, bei der ein Strahldispergator mit einander gegenüberliegenden Kapillarbohrungen verwendet wird. Die durch diese Kapillarbohrungen austretenden Emulsionsstrahlen treffen dadurch aufeinander. Änderungen der Verfahrensparameter sind wegen der nicht variablen Kapillarbohrungen hier nur durch Austausch des gesamten Strahldispergators möglich.

Aufgabe der vorliegenden Erfindung ist es, ein Homogenisierverfahren bereitzustellen, das die Nachteile des Standes der Technik vermeidet, einfach zu handhaben ist und nicht auf extreme Verfahrensbedingungen angewiesen ist. Erfindungsgemäss wird diese Aufgabe dadurch gelöst, dass man die zu homogenisierende Emulsion in zwei Teilströmen aus zwei gegeneinander gerichteten, miteinander genau fluchtenden und im geringen Abstand voneinander angeordneten Düsen in eine Homogenisierkammer eindüst, wobei die Düsenaustrittsgeschwindigkeit jedes Teilstroms mindestens 75 m/s beträgt.

Soweit hier und in der folgenden Beschreibung von Emulsionen die Rede ist, sollen damit alle Zustände der Mischung an sich nicht mischbarer Flüssigkeiten bezeichnet werden, bei denen eine weitergehende Aufteilung der emulgierten Phase im Emulsionsmittel, also eine weitere Homogenisierung, gewünscht wird.

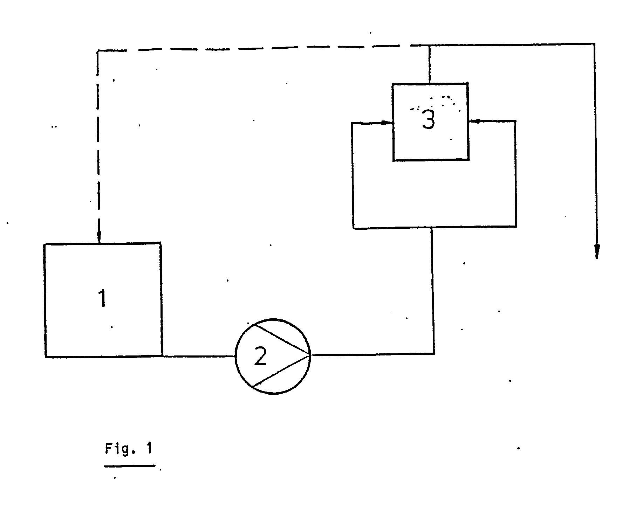

Praktisch wird das erfindungsgemässe Verfahren, wie anhand des Fliess-Schemas der Figur 1 erläutert wird, in der Weise durchgeführt, dass man die zu homogenisierende Emulsion aus einem Vorratsbehälter (1) durch eine geeignete Pumpe (2) in zwei sich hinter der Pumpe teilenden, gleichgrossen Teilströmen zu der Homogenisierkammer (3) pumpt und durch die in dieser Kammer angebrachten Düsen mit den Austrittsstrahlen der Teilströme aufeinandertreffen lässt. Dabei ist es nicht erforderlich, dass die Homogenisierkammer stets völlig leer ist, vielmehr können die Teilströme auch in eine mit schon eingedüster Emulsion gefüllte Kammer eingedüst werden. Falls erwünscht, kann die Emulsion ganz oder teilweise für eine erneute Passage dem Vorratsbehälter (1) oder einem gesonderten Behälter zugeführt werden.

Die für eine Homogenisierung notwendige Düsenaustrittsgeschwindigkeit der Teilströme liegt im Bereich von oberhalb 70 m/s. Die zur Erzielung dieser Geschwindigkeiten erforderlichen Drücke liegen in Abhängigkeit von der Düsengeometrie oberhalb von etwa 130 bar. Der Abstand der Düsenöffnungen hängt von der Grösse der Düsenöffnungen ab und kann durch einfache Versuche leicht optimiert werden. Ist der Abstand der Düsenöffnungen zu klein, so tritt ein Rückstau ein, weil die eingedüste Emulsion nicht schnell genug seitlich ausweichen kann. Dies muss auf jeden Fall vermieden werden. Bei einer Düsenöffnung von 0,2 mm muss der Düsenabstand mindestens 2 mm betragen. Beträgt der Durchmesser der Düsenöffnung 1 mm, so ist ein Düsenabstand in der Grössen ordnung von etwa 60 mm notwendig.

Der Grad der Homogenisierung der Emulsion hängt entscheidend von der Art der Emulsion und von der Düsenaustrittsgeschwindigkeit ab.

Wenn im Einzelfall der Druck bzw. die Austrittsgeschwindigkeit der Teilströme für eine ausreichenden Homogenisierung bei einmaligem Durchgang nicht ausreicht, ermöglichen Mehrfachpassagen eine deutliche Verbesserung der Ergebnisse.

Die Verfahrensoptimierung für die im Einzelfall gewünschte Homogenisierung im Rahmen der erfindungsgemässen Parameter des Verfahrens und Vorrichtungsmerkmale liegt im Bereich des Fachwissens und erfordert nur einfache Versuche.

## Ansprüche

1. Verfahren zur Homogenisierung von Emulsionen, dadurch gekennzeichnet, dass man die Emulsion in zwei gleichgrossen Teilströmen durch zwei gegeneinander gerichtete, miteinander fluchtende und in geringem Abstand voneinander angeordnete Düsen mit einer Düsenaustrittsgeschwindigkeit von je mindestens 75 m/s in eine Homogenisierkammer eindüst.

2. Verfahren nach Anspruch 1, dadurch gekennzeichnet, dass die Durchflussgeschwindigkeit der Teilströme 80 - 300, vorzugsweise 100 - 200 m/s beträgt.

3. Verfahren nach Anspruch 1 oder 2, dadurch gekennzeichnet, dass die Emulsion mehrfach durch die Düsen geleitet wird.

4. Vorrichtung zur Durchführung des Verfahrens nach Anspruch 1 - 3, bestehend aus

(a) einem Vorratsbehälter für die zu homogenisierende Emulsion,

(b) einer Förderpumpe zum Transport der zu homogenisierenden Emulsion,

(c) einer Homogenisierkammer mit zwei gegeneinander gerichteten, genau miteinander fluchtenden und in geringem Abstand voneinander im Inneren der Kammer angeordneten Düsen,

(d) einer Verbindungsleitung von dem Vorratsbehälter zu der Förderpumpe und von der Förderpumpe zu den beiden Düsen,

(e) einer Entnahmestelle für die homogenisierte Emulsion und

(f) gegebenenfalls einer Rückflussleitung von der Homogenisierkammer zum Vorratsbehälter.

5. Vorrichtung nach Anspruch 4, dadurch gekennzeichnet, dass die Düsen eine Öffnung mit einem Durchmesser von 0,2 bis 1,0 mm aufweisen und in Abhängigkeit von dem Durchmesser der Düsenöffnung in einem Abstand von 2 bis 60 mm angeordnet sind.

Fig. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 533 254 (COOK) * Spalte 5, Zeile 49 - Spalte 6, Zeile 54; Spalte 8, Zeilen 3-32; Figuren * --- | 1-5 | B 01 F 5/02 |
| A | US-A-2 751 425 (RUPP) * Spalte 9, Zeilen 13-23; Figuren * --- | 5 | |
| A | GB-A-2 063 695 (KONISHIROKU) * Seite 1, Zeilen 100-104; Figuren * --- | 5 | |
| A | US-A-2 976 024 (MARTINEK) --- | | |
| A | US-A-2 751 335 (CARVER) --- | | |
| A | US-A-3 391 908 (MacDONALD) ----- | | |

RECHERCHIERTE SACHGEBIETE (Int. Cl.4)

B 01 F

Der vorliegende Recherchenbericht wurde für alle Patentansprüche erstellt

| Recherchenort | Abschlußdatum der Recherche | Prüfer |
|---|---|---|
| DEN HAAG | 23-06-1989 | PEETERS S. |